# EUROPEAN PATENT SPECIFICATION

(11) **EP 0 837 120 B1**
(45) Date of publication and mention of the grant of the patent: **24.07.2002**
(21) Application number: 97117245.7
(22) Date of filing: 06.10.1997
(51) Int. Cl.: C10J 3/00, C07C 1/00, C10J 3/14, C10J 3/26

(54) **Perfected method of fuel gasification and relative gasifier device**
Verbesserte Methode zum Vergosen von Brennstoff und entsprechende Vergosungsvorrichtung
Méthode perfectionnée pour la gazéification de combustible et dispositif s'y rapportant

(30) Priority: 16.10.1996 IT UD960196
(43) Date of publication of application: 22.04.1998
(73) Proprietor: SAS GINO TOMADINI & C., I-33170 Pordenone (IT)
(72) Inventor: Tomadini, Gino, 33170 Pordenone (IT)
(74) Representative: Petraz, Gilberto Luigi

(56) References cited:
- EP-A- 0 259 927
- DE-A- 3 505 429
- GB-A- 2 158 841
- US-A- 5 019 135

## Description

This invention concerns a perfected method for the gasification of fuel and also the relative gasifier device as set forth in the respective main claims.

The invention is applied in the production of combustible gases from solid urban and/or industrial waste, or low-cost solid products such as wood scraps or chippings, solid biomasses in general, peat, lignite, carbon fossiles and other products, possibly integrated or combined with liquid or gaseous fuels.

The state of the art covers a plurality of methods and devices, commonly called gasifiers or also gas generators, used to produce combustible gases from scraps of various types or low cost raw materials.

Combustible gases thus produced are used to power machines of various types such as for example engines for generator sets to produce electric energy.

Gasification technology for the production of combustible gas starting from carbons, peat and lignite has been known for some time, but has recently been developed for the use of combustible raw materials which are more difficult to process, such as solid urban and/or industrial waste. This is both in order to reduce environmental pollution and also to compensate for the lack of main energy sources. The gasification of solid urban refuse or industrial waste with methods known to the state of the art has however often proved inefficient both due to problems connected directly to the gasifier and also problems arising during the various steps of the gasification process.

A first disadvantage is that these methods are highly polluting for the environment, as they do not allow all types of refuse to be gasified; to be more exact, it is inadvisable to use industrial waste containing toxic substances or those which are difficult to dispose of, for example wood scraps impregnated with resins and/or paint.

A further disdvantage which operators in this field particularly complain of is that devices known to the state of the art provide only low-grade combustible gases and therefore, in most applications, they do not give satisfactory energy yields.

Moreover, known methods give a low yield in that they require large quantities of solid fuel to produce a sufficient volume of combustible gas to supply the user devices. In order to increase this yield, some have tried to recover the heat of the combustible gases leaving the gasifier to pre-heat the comburent fluids, but the heat generated by the user devices has never been exploited and the exhaust gases produced by these devices are dispersed directly into the atmosphere with considerable damage to the environment.

Therefore, in methods known to the art, only 40% of the energy supplied to the user device by the combustible gas is transformed into usable energy while the remaining 60% is dispersed with the burnt gases and in the cooling of the user device itself.

A further disadvantage is that known gasifiers cannot easily use solid fuels of a heterogeneous type and/or which have a disuniform granulometry. In such cases in fact, the descent of the solid fuel into the gasifier is not uniform and causes gaps and/or bridges to appear in the material which are particularly harmful for the gasification process.

Moreover, methods known to the art allow solid fuels or liquid fuels to be used, but a combined and simultaneous use of the two types of fuel is not possible; however this would bring considerable advantages both in terms of the quality of the combustible gas produced and also from the environmental and economic point of view.

The present applicants have designed, tested and embodied this invention to overcome the shortcomings of the state of the art, and to achieve further advantages.

This invention is set forth and characterised in the respective main claims, while the dependent claims describe variants of the idea of the main embodiment.

The purpose of the invention is to provide a gasification method able to increase the yield of the relative gasifier.

A further purpose is to provide a method able to offer a plurality of points of intervention intended both to improve and/or to control the characteristics of the gas produced and also to reduce environmental pollution.

A further purpose is to produce a high-grade combustible gas with a higher calorific value per unit of measurement than that produced by methods known to the state of the art.

A further purpose is to enable a high recovery of energy and particularly the controlled re-use of the gases produced by the user devices such as for example the exhaust gases generated by the engines or steam boilers of the generator sets.

Another purpose is to enable an efficient use of solid fuels of different types, including heterogeneous types and/or those with a disuniform granulometry.

A further purpose is to enable solid fuels to be used which are difficult to dispose of, such as waste containing toxic and/or harmful substances such as resins and paints, thus embodying an efficient damping of all the pollutants, rendering them harmless before they are released into the environment.

According to the method according to the invention, there is a prior dry distillation, by means of pre-heating, of the volatile parts of the solid fuels immediately after the introduction of the solid fuel into the gasifier, which enables the volatile parts to be sent directly for oxidising combustion so as to produce a combustible gas without any tarry substances.

This also makes it possible to accelerate carbonisation or coking of the solid fuels, thus facilitating, as they are gasified separately, a more regular process, in sequence, of the oxidising combustion, where carbon dioxide and hydrogen without water vapour is produced, and of reducing combustion, where the carbon dioxide turns into carbon monoxide.

According to the invention, gasification is assisted by the fact that the fluids moving inside the gasifier are free to follow both ascending/descending routes in a vertical direction, and also horizontal routes; this makes it possible to accelerate the gasification process and to use solid fuel of any granulometry whatsoever and/or with different granulometry.

According to the invention, the aforesaid horizontal routes of the fluids inside the gasifier are achieved by the inclusion of walls with holes in them, suitably arranged inside the gasifier.

According to the invention, at least the walls with holes arranged in the area of the gasifier affected by the reducing combustion are made of nickel alloy or treated with nickel.

Nickel exerts a catalysing action on the carbon and free hydrogen produced by the reducing combustion, assisting the formation of methane gas and the production of a quantity of heat such as to require a lesser quantity of comburent air and therefore allow the production of a combustible gas with a low percentage of azote.

The formation of methane gas is assisted by the moderate temperature and speed, which tend to decrease, of the gas in formation which passes from the central zone, where the oxidising combustion takes place, to the peripheral zone, where the reducing combustion takes place.

The method according to the invention exploits to the maximum energies which otherwise would be lost, such as the heat possessed by the gas produced, the cooling heat of the mechanical parts of the user devices, the heat of the exhaust gases of the user devices and other energy.

According to the invention, at least one of these sources of heat, or more than one in combination, is used in order to achieve the following functions:
- dry distillation of the solid fuel;
- evaporation and superheating of any liquid fuel;
- dessication/drying and/or dehydration of the solid fuel which is to be loaded;
- evaporation and/or superheating of the comburent fluids.

In accordance with the invention, these sources of heat are used by heat exchangers according to the percentage of humidity and/or of volatile parts contained in the fuel.

In the preferred embodiment according to the invention, the heat possessed by the combustible gas produced, and/or by the exhaust gases of the user devices, is used by at least a first heat exchanger used to dry any wet solid fuel and/or by at least a second heat exchanger used to produce water vapour to be used as a comburent and/or by at least a third heat exchanger used to superheat the comburent water vapour and air and/or by at least a fourth heat exchanger used to transform the liquid fuels into vapour and/or by at least a fifth heat exchanger used to superheat the vapours of the liquid fuel.

According to the invention, at least the fifth heat exchanger is embodied with an interspace placed on the periphery of the gasifier device in order to exploit the heat inside the latter so as to superheat the vapours of the liquid fuel added to the distilled volatile parts of the solid fuel.

According to the invention, moreover, the exhaust gases produced by the machines are used as additional fluid for the comburent fluids in order to automatically control the calorific value of the combustible gas produced.

Also according to the invention, before being sent to the user devices, the combustible gases produced are purified of the suspended particles, acids and all pollutants and/or substances unsuitable for the correct functioning of the machines.

The same applies to the gases produced in combustion by the machines and sent to the various heat exchangers and particularly after these gases have been used to dry those fuels containing pollutant substances.

In the preferred embodiment of the invention, this purification is achieved by means of water washing and then passing the gas through granulated calcium carbonate (CaCO₃).

In this embodiment, the washing water is continuously purified of the suspended particles and the toxic or harmful substances damped by the gas, by means of mechanical and/or chemical systems.

According to the invention, at least at the inlet of the heat exchangers used to transform the water into vapour, there are descaling units to remove at least the calcium from the water so as to prevent scaling in the pipes of the exchanger.

The attached drawings are given as a non-restrictive example and illustrate a preferred embodiment of the invention as follows:
- Fig.1: shows a block diagram of the method according to the invention;

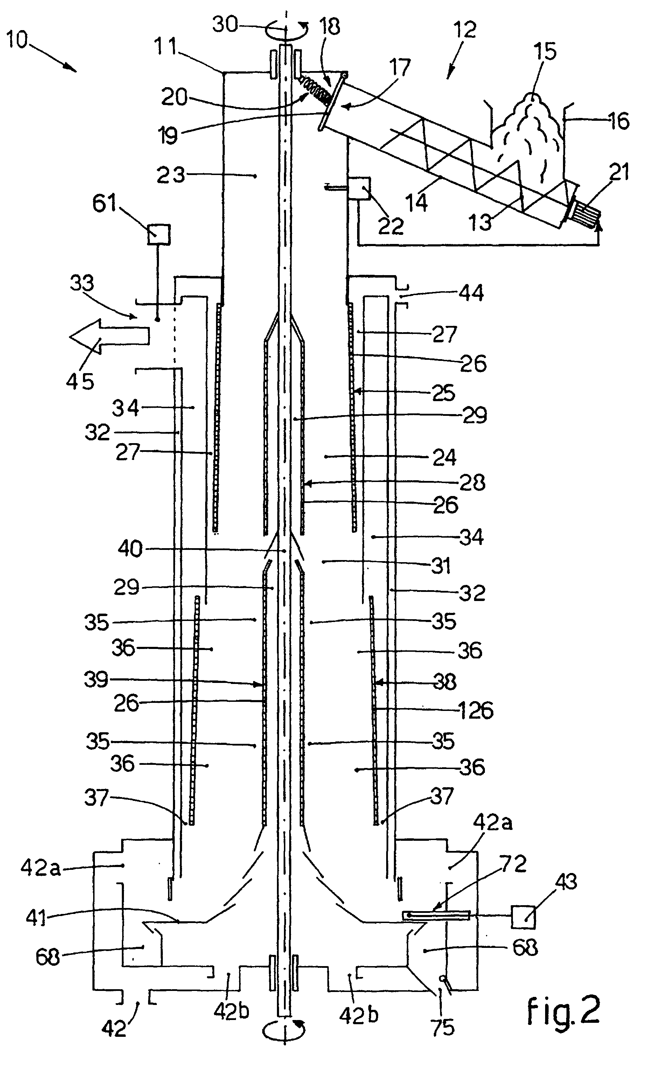

- Fig.2: shows in diagram form a gasifier device according to the invention.

The gasifier 10 according to the invention includes a containing structure 11 on a vertical axis 30, substantially cylindrical in shape and equipped with the appropriate insulating covering at least on the outer peripheral surface.

The containing structure 11 is equipped at its upper part with loading means 12, of the gas-tight type, for the solid fuel 15.

In this case, the loading means 12 consist of a metering screw 13 coaxial to a containing cylinder 14, which loads the solid fuel 15 which descends through the loading mouth 16 and pushes the fuel into the gasifier 10.

According to a variant, the loading means 12 consist of a hopper or other loading device known to the state of the art, provided it is gas-tight.

The metering screw 13 is, in this case, shorter than the containing cylinder 14 so that the solid fuel 15 forms, in correspondence with the upper end 17 of the containing cylinder 14, a plug of material which prevents the leakage of the combustible gas produced from the gasifier 10 and also the inlet of cold air through the loading mouth 16; this condition is also ensured by the fact that the containing cylinder 14 is inclined downwards with respect to the right angle of the vertical axis 30 of the gasifier 10 so that its upper end 17 is higher than the loading mouth 16.

Moreover, so as to ensure an air-tight condition with respect to the outer environment, the upper end 17 of the containing cylinder 14 is equipped with closing means 18, in this case consisting of a door element 19 hinged onto the upper end 17, so as to divert downwards the solid fuel 15 thrust by the metering screw 13 and direct it into the loading area 23.

According to one possible embodiment, the door element 19 closes the upper end 17 because of its own weight; according to the variant shown here, closure is obtained by means of elastic means 20 such as for example a spring, a piston or other means.

The metering screw 13 is actuated by motor means 21 controlled by means to monitor the level 22 which govern the activation of the metering screw 13 so as to ensure the continual presence in the gasifier 10 of a desired quantity of solid fuel 15.

The solid fuel 15, unloaded into the loading area 23, begins to heat up due to the effect of the hot gaseous fluids which, rising from the lower part of the gasifier 10, replace the colder ones present around the loaded solid fuel 15, thus causing a circling motion of the gaseous fluids with a substantially vertical trend.

To be more exact, the cold gaseous fluids tend to descend into the underlying zone 24 for the dry distillation of the volatile parts of the solid fuel 15.

In this dry distillation zone 24, the solid fuel 15 is enclosed at the sides by an upper outer containing wall 25 and an upper inner containing wall 28, both of which include through holes 26; the holes allow the colder gaseous fluids of the solid fuel 15 to be exchanged with the hotter gaseous fluids arriving from the intermediate zone 31, with the fluids circulating in the outer interspace 27 superheated by the heat of the combustible gas 45 flowing in the interspace 34 and with those at the dry distillation stage in the zone 24; the holes also allow the fluids of the interspace 24 to be exchanged with those of the inner interspace 29.

In this way therefore, apart from the interchange of hot, rising gaseous fluids with cold gaseous fluids descending with a vertical motion, the gasifier 10 also allows an interchange of these gaseous fluids in a horizontal sense through the through holes 26.

In other words, the volatile parts of the fuel 15 distilled in the dry distillation zone 24 pass through the through holes 26 in the interspace 27, from here they flow into the interspace 32 and follow it with a descending motion towards the lower part of the gasifier 10, superheating during their journey as a consequence of the extreme heat of the combustible gas 45 produced, flowing towards the outlet door 33 through the interspace 34.

The superheating of the volatile parts, apart from in the interspace 32, therefore begins in the interspace 27 since this zone too is adjacent to the interspace 34 which is lapped by the combustible gas 45 produced which, given the high temperature of the latter, yields heat to the dry distillation of the volatile parts of the solid fuels 15 present in the zone 24.

The distilled volatile parts follow this interspace 32, reach the lower part of the gasifier 10 and feed the innermost oxidising zone 35 where they burn completely.

The gases produced in this zone 35 pass to the more outerly zone 36, with its reducing action, and are then aspirated into the interspace 34, through the through holes 126 of the lower outer containing wall 38; at the same time, the gas produced by the oxidising and reducing combustion of the distilled volatile parts of the fuels 15 flows through the circumferential aperture 37.

The circumferential aperture 37 also serves to make any heavy particles present in the gaseous fluids circulating in the interspace 34 precipitate to the lower part of the gasifier 10, thus preventing these particles from reaching the outlet door 33.

The solid fuel 15 passes from the dry distillation zone 24 to the intermediate zone 31 and from there, reduced now to its carbon only state, descends for gasification both to the oxidising combustion zone 35 and to the reducing zone 36.

The gaseous fluids produced flow from the oxidising zone 35 to the reducing zone 36 where they intensify and, with a horizontal, tendentially ascendant motion, pass through the through holes 126 of the lower outer containing wall 38 and enter the interspace 34.

The lower outer containing wall 38, in cooperation with the lower inner containing wall 39, like the aforesaid walls 25 and 28, has the function of retaining the solid fuel 15 at the side, and also to allow the passage of the gaseous fluids in a substantially horizontal direction.

The lower outer containing wall 38 is in this case embodied in nickel alloys, or at least treated on its inner surface with nickel, in such a way as to function as a catalyser for the free carbon and hydrogen and assist the formation of methane gas.

The conditions necessary for the formation of methane gas are assisted by the fact that the gas as it forms and passes from zone 35 to zone 36 is at a temperature and speed which are progressively decreasing.

The gasifier 10 includes, in correspondence with the central axis 30, a rotary shaft 40 able to rotate on its own axis when it is actuated by the appropriate motor means, which are not shown here.

Solidly associated with the rotary shaft 40 are both the upper inner wall 28 and the lower inner wall 39 which includes at its lower part a progressive enlargement of cross section so as to be associated at its end with a disk element 41 to collect and discharge the ashes.

In this case, the ashes are discharged by means of at least one inclined blade element 72 which is suitably activated when a temperature sensor 43 detects that the temperature of the ashes is suitable for their expulsion into an appropriate discharge chamber 68 including at least one aperture 75 for the ashes to be expelled out of the gasifier 10.

The rotation of the upper inner wall 28 and lower inner wall 39 assists the sequential and continuous descent of the solid fuel 15 from the loading zone 23 to the underlying zones until it reaches the disk element 41 in the form of ash, avoiding the formation of harmful bridges and discontinuity in the solid fuel 15.

The uniform and continuous descent of the solid fuel 15 is also assisted by the shape, which is like a truncated cone with the larger base facing downwards, of both the upper outer wall 25 and the lower inner wall 38.

The comburent fluids such as air 58, initially heated in the heat exchanger 48, and water vapour 59 produced by the heat exchangers 47 and 62, and also by the dehydrator 57, which are necessary for the production of the combustible gas 45, are superheated in advance, in the heat exchanger 46, by the heat of the combustible gas 45 as it leaves the interspace 34 and/or the heat developed by the machines 54 fed by the combustible gas 45 produced; these fluids are fed into the gasifier 10 through the conduit 42 and they spread towards an aperture 42a, to feed the combustion of the volatile parts of the solid fuel 15, and towards an aperture 42b, to feed the combustion of the carbon parts of the solid fuel 15.

This conduit 42 also receives the exhaust gases produced by the machines 54 such as, for example, motors or steam boilers for generating sets, so as to control the calorific value of the combustible gas 45 produced and adapt it to the type and the characteristics of the motor it must drive.

The gasifier 10 also includes an inlet mouth 44 where are introduced the vapours of the liquid fuels which are superheated and mix with the volatile parts of the solid fuels 15 in the interspace 32.

The gasifier 10 includes at least one thermostat 61 arranged on the outlet mouth 33 so as to automatically control the temperature of the combustible gas 45 produced and automatically modulate the percentage of comburent air 58 and vapour 59 to be fed into the gasifier 10 so as to obtain combustible gas 45 with the desired temperature, in order to render the process of gasification more regular and to improve the overall efficiency of the whole system.

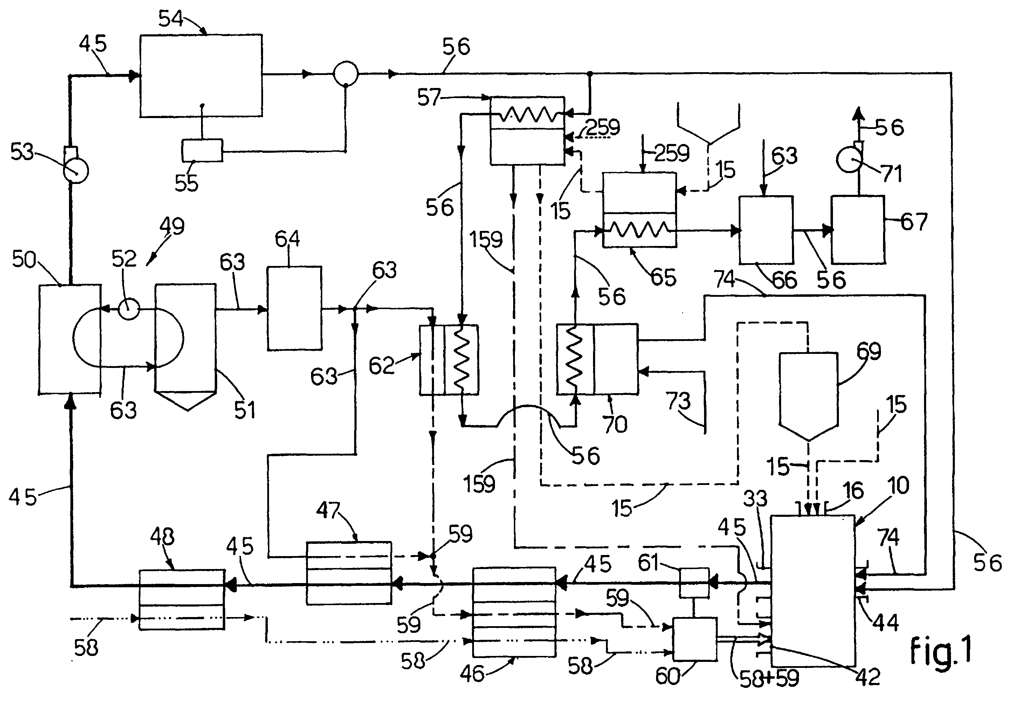

The block diagram shown in Fig.1 shows how the combustible gas 45 produced by the gasifier 10 is partly cooled by the heat exchange caused by the dry distillation of the volatile parts of the fuel 15, by the superheating of the vapours of the liquid fuels introduced into the gasifier 10 through the appropriate inlet mouth 44 and by the heat exchange achieved by a plurality of heat exchangers.

In this case, there is a first heat exchanger 46 which superheats the comburent fluids.

The combustible gas 45 is then cooled further as it passes through a second heat exchanger 47 and afterwards in a third heat exchanger 48 to which it yields its own heat, respectively for the production of water vapour 59 used as a comburent and for the pre-heating of the comburent air 58.

At this point the combustible gas 45 passes through a washing device 49 whose function is to purify it of the suspended powders, acid substances and any other polluting elements present and, at the same time, to cool it to the desired temperature.

In this case, the washing device 49 consists of a device 50 containing calcium carbonate (CaCO₃) into which is introduced washing water fed in a continuous circle, by means of a pump 52, from a shower-type cooling tower 51 which both cools the water 63 and decants from it the solid parts which have been damped down.

In the shower-type cooling tower 51 it is also possible to perform other interventions of a mechanical and/or chemical nature intended to damp down any polluting substances dissolved in the washing water 63; the recycling of the water 63 is achieved by continuously sending part of the water 63 to evaporate at least in the heat exchangers 47 and 62.

Once it has been washed and cooled, the combustible gas 45 leaving the device 50, assisted by a fan 53 able to make it overcome the resistances met with on its passage through the conduits and various elements and/or devices in the process, is sent to feed a machine 54 consisting of the motor or steam boiler of a generating set.

In certain cases of particular use, in cooperation with the motor there is a thermostat 55 which controls the temperature and automatically arranges the supply of the exhaust gases 56 to the gasifier 10, in such a way that they partly substitute the comburents - air 58 and water vapour 59 - and thus increase the quantity of inert azote, and decrease the calorific value of the combustible gas 45 produced.

The exhaust gases 56, are fed not only to the gasifier 10 but also to a heat exchanger 57 for the dehydration of the wet solid fuels 15; the vapour 159 generated by the dehydration and the fuel 15 thus dehydrated are both sent to the gasifier 10; in this case, the dehydrated solid fuel 15 is supplied to the gasifier 10 by means of a silos 69 which functions as a supply and storage zone.

The comburent fluids 58 and 59 are automatically mixed and proportioned in appropriate regulator units, in this case composed of valves 60 automatically governed by the thermostat 61 according to the temperatures possessed by the combustible gas 45 as it leaves the gasifier 10.

In order to dehydrate the solid fuel and produce vapour, the exhaust gases 56 are sent from the heat exchanger 57 to another heat exchanger 62 whose function is to make the water 63 evaporate so as to produce the water vapour 59 which is superheated in the heat exchanger 46.

From the heat exchanger 62, the exhaust gases 56 pass to another heat exchanger 70 whose function it is to transform the liquid fuels 73, for example solvents contained in the paint sludges, into vaporised fuel 74 which is sent to the inlet mouth 44 of the gasifier 10.

The surplus water vapour 259 produced and/or not needed by the gasifier 10 for the production of combustible gas 45, is fed to a drying device 65 which is also fed by the exhaust gases 56 and used to obtain a pre-drying of the solid fuel 15.

In certain particular applications, the water vapour 259 is fed also to the heat exchanger 57 to assist with the final dehydration of the solid fuel 15.

This device 65 is used especially in the case of very damp solid fuels 15 such as, for example, bio-masses.

Before being discharged into the atmosphere, by means of a centrifugal ventilator 71 which is able to make them overcome the resistances which they meet on their way, the exhaust gases 56 from the dehydrating device 65 pass through a cooler 66, in this case of the shower type using water 63, which, apart from lowering their temperature, purifies them of the suspended vapours and powders. There is also a device 67 to eliminate the unpleasant odours of the exhaust gases, used particularly if the solid fuel is composed prevalently of solid urban waste.

The water 63 used to damp down the polluting substances in the cooling tower 51 and the water 63 used in the shower-type cooler 66, before being sent to the heat exchangers 47 and 62, undergo a treatment 64, in this case of the magnetic type, intended to damp down the calcium content so as to prevent scaling in the pipes of the heat exchangers 47 and 62.

## Claims

1. Method for the gasification of solid fuels (15) to produce combustible gas (45) starting from solid urban and/or industrial waste, wood scraps and/or chippings, biomasses in general, carbons, peat, or lignite, said method using at least a gasifier associated at least with a device (54), consisting of a steam motor or boiler, able to use the combustible gas (45) produced, the method being **characterised in that** it provides to perform, outside said gasifier (10), alternately or jointly, at least a procedure of thermal recovery of at least part of the heat possessed by said combustible gas (45) emerging from the gasifier (10), of at least part of the heat possessed by the exhaust gases produced by said user device (54), or of at least part of the heat generated by the mechanical parts of said user device (54), the method also being **characterised in that** it provides a prior distillation, by means of pre-heating, of the volatile parts of the solid fuel (15) immediately after the introduction of the solid fuel (15) into said gasifier (10) to produce a combustible gas without any tarry substances, and provides to include, in the lower part of said gasifier (10), catalyser means made of nickel able to assist the formation of methane gas (45), and **in that** in an upper part (24) of the gasifier (10) it provides an exchange in both a vertical and horizontal direction between descending cold fluids and/or distillates of the volatile parts present in the combustible material (15) loaded from above and the hot fluids ascending from the intermediate and lower part of the gasifier (10) so as to obtain in said upper part (24) a dry distillation due to heating without combustion of the volatile parts of said solid fuel (15), said gasifier (10) including inner walls (28, 39) contained within outer walls, respectively upper (25) and lower (38), said walls (25,28,38,39) defining substantially annular channels through which the fuel (15) descends and including through holes (26,126) for the passage and/or at least horizontal interchange of the descending cold gaseous fluids of the solid fuel (15) and the ascending hot gaseous fluids circulating in coordinated interspaces (27,34).

2. Method as in Claim 1, **characterised in that** it provides that a desired part of the exhaust gases (56) produced by said user device (54) is re-introduced into the gasifier (10), together with comburent fluids (58-59), in order to reduce the calorific power of the combustible gas (45) produced.

3. Method as in Claim 1, **characterised in that** at least part of the heat possessed by the combustible gas (45) is used, in at least an exchanger (46, 48) outside the gasifier (10), to heat comburent air (58) before said air (58) is sent inside the gasifier (10).

4. Method as in Claim 1, **characterised in that** at least part of the heat possessed by the combustible gas (45) is used, in an exchanger (46, 47) outside the gasifier (10), to heat water vapour (59) before said water vapour (59) is sent inside the gasifier (10).

5. Method as in Claim 1, **characterised in that** at least part of the heat possessed by the exhaust gases (56) of said user device (54) is used, in a heat exchanger (57), to previously dehydrate said solid fuels (5) before they are sent inside the gasifier (10).

6. Method as in Claim 1, **characterised in that** at least part of the heat possessed by the exhaust gases (56) of said user device (54) is used, in a heat exchanger (62), to obtain water vapour (59) to send inside the gasifier (10).

7. Method as in Claim 1, **characterised in that** at least part of the heat possessed by the exhaust gases (56) of said user device (54) is used, in a heat exchanger (70), to transform liquid fuels (73) into vaporised fuel (74) to be sent inside the gasifier (10).

8. Method as in Claim 1, **characterised in that** at least part of the heat possessed by the exhaust gases (56) of said user device (54) is used, in a drier device (65), to perform a previous drying of the solid fuel (15) to be sent inside the gasifier (10).

9. Method as in any claim hereinbefore, **characterised in that** it provides to use at least one washing device (49) to purify the combustible gas (45) produced before it is fed to the user device (54).

10. Method as in Claim 9, **characterised in that** the washing device (49) comprises a device (50) containing granulated calcium carbonate (CaCO₃) sprayed with water (63) for washing the gas (45), the water (63) being supplied from a shower-type cooling tower (51).

11. Method as in Claim 10, **characterised in that** the shower-type cooling tower (51) provides to purify the washing water by means of mechanical and/or chemical systems.

12. Method as in any claim hereinbefore, **characterised in that** it provides to use anti-scaling means (64) of at least the water (63) which is to be transformed into vapour (59).

13. Method as in any claim hereinbefore, **characterised in that** the exhaust gases (56), before being expelled into the atmosphere, are cooled, purified and deprived of unpleasant odours and of polluting substances by means of a purifying-cooling device (66) and/or a device to remove odours (67).

14. Apparatus for the production of combustible gas (45) starting from solid fuels (15), such as solid urban and/or industrial waste, wood scraps and/or chippings, bio-masses in general, carbons, peat, or lignite, said apparatus comprising at least a gasifier and at least a user device (54), consisting of a steam motor or boiler, of said combustible gas (45), the gasifier including loading means (12) for the solid fuel (15) in an upper loading zone (23) located above at least an upper dry distillation zone (24) and at least a lower reducing zone (36) cooperating with a contiguous oxidising zone (35), the device being **characterised in that** said gasifier includes inside at least a pair of inner walls (28,39) to contain the solid fuel (15), the walls being contained within a pair of outer walls, respectively the upper outer wall (25) and the lower outer wall (38), said walls (25,28,38,39) defining substantially annular channels through which the fuel (15) descends and including through holes (26,126) for the passage and/or at least horizontal interchange of the descending cold gaseous fluids of the solid fuel (15) and the ascending hot gaseous fluids circulating in coordinated interspaces (27,34) defined at least outside said outer walls (25, 38), and **in that** outside said gasifier (10) the apparatus comprises heat exchangers (46, 47, 48) able to recover at least part of the heat possessed by the combustible gas (45) produced and by the exhaust gases of said user device (54) to heat at least comburent fluids (58, 59) to be sent to said gasifier (10).

15. Apparatus as in Claim 14, **characterised in that** said outer containing walls (25,38) of said gasifier (10) define a volume shaped like a truncated cone with its larger base facing towards the bottom of the gasifier (10) and said inner containing walls (28,39) are able to be made to rotate around a central axis (30) of the gasifier (10).

16. Apparatus as in Claim 14, **characterised in that** at least said lower outer containing walls (38) placed near the reducing zone (36) are embodied in nickel alloy, or lined at least inside with nickel.

17. Apparatus as in Claim 14, **characterised in that** the lower inner wall (39) and the upper inner wall (28) of said gasifier (10) are solid with a rotary shaft (40).

18. Apparatus as in any claim from 14 to 17 inclusive, **characterised in that** said gasifier (10) includes at the lower part a disk element (41) to collect and expel the ashes, the disk element (41) being placed in cooperation with at least a blade element (72) for the controlled discharge of the ashes.

19. Apparatus as in Claim 18, **characterised in that** said gasifier (10) includes a sensor device (43) to monitor the temperature at least of the ashes whose discharge it governs.

20. Apparatus as in any claim from 14 to 19 inclusive, **characterised in that** said gasifier (10) includes gas-tight loading means of the metering screw type (13).

21. Apparatus as in Claim 20, **characterised in that** the metering screw means (13) are contained in a cylinder (14) inclined downwards with respect to the right angle of the axis (30) of the gasifier (10).

22. Apparatus as in any claim from 20 to 21 inclusive, **characterised in that** the metering screw means (13) are driven by motor means (21) governed by sensor means to monitor the level (22).

23. Apparatus as in Claim 14, **characterised in that**, outside the gasifier (10), it comprises at least a heat exchanger (57) to previously dehydrate the fuel (15) by sending the hot exhaust gases (56) produced by the user device (54) into said exchanger (57).

24. Apparatus as in Claim 14, **characterised in that**, outside the gasifier (10), it comprises at least a heat exchanger (64) to obtain water vapour (59) by sending the hot exhaust gases (56) produced by the user device (54) into said exchanger (62).

25. Apparatus as in Claim 14, **characterised in that**, outside the gasifier (10), it comprises at least a heat exchanger (70) to transform liquid fuels (73) into vaporised fuels (74) by sending the hot exhaust gases (56) produced by the user device (54) into said exchanger (70).

26. Apparatus as in Claim 14, **characterised in that**, outside the gasifier (10), it comprises at least a washing device (49) to wash said combustible gas (45) with the function of purifying it of the powders present in suspension, acid substances and other pollutant elements.

27. Apparatus as in Claim 26, **characterised in that** said washing device (49) comprises at least a device (50) which washes with calcium carbonate.

## Patentansprüche

1. Verfahren zur Vergasung von festen Kraftstoffen (15) unter Bildung eines brennbaren Gases (45), welches von einem festen Haushalts- und/oder Industrieabfall, Holzresten und/oder - schnitzeln, Biomasse im allgemeinen, Kohlenstoffen, Torf oder Lignit ausgeht, wobei das Verfahren mindestens einen Vergaser einsetzt, der mit mindestens einer Vorrichtung (54) verbunden ist, die aus einem Dampferzeuger oder Boiler besteht und das hergestellte brennbare Gas (45) verwenden kann, wobei das Verfahren **dadurch gekennzeichnet ist, daß** es außerhalb des Vergasers (10) abwechselnd oder gemeinschaftlich mindestens ein Verfahren zur Wärmegewinnung mindestens eines Teils der Wärme des brennbaren Gases (45), das aus dem Vergaser (10) austritt, mindestens eines Teils der Wärme des durch die Verbrauchervorrichtung (54) erzeugten Abgases oder mindestens eines Teils der durch die mechanischen Teile der Vorrichtung (54) erzeugten Wärme bereitstellt, wobei das Verfahren auch **dadurch gekennzeichnet ist, daß** es eine Vordestillation durch Vorerhitzen der flüchtigen Teile des festen Kraftstoffs (15) unmittelbar nach der Einführung des festen Kraftstoffs (15) in den Vergaser bereitstellt, um ein brennbares Gas ohne jegliche teerartige Substanzen herzustellen, und daß in dem unteren Teil des Vergasers (10) Katalysatormittel aus Nickel, die zur Bildung von Methangas (45) beitragen, enthalten sind, und daß in einem oberen Teil (24) des Vergasers (10) ein Austausch sowohl in vertikaler als auch horizontaler Richtung zwischen den absteigenden kalten Fluiden und/oder den Destillaten der flüchtigen Teile, die in dem brennbaren Material (15), das von oben eingespeist wird, enthalten sind, und den heißen Fluiden, die von dem mittleren und unteren Teil des Vergasers (10) aufsteigen, ermöglicht wird, so daß in dem oberen Teil (24) aufgrund des Erhitzens eine Trockendestillation ohne Verbrennung der flüchtigen Teile des festen Kraftstoffs (15) erzielt wird, wobei der Vergaser (10) innerhalb der äußeren Wände, jeweils eine obere Wand (25) und eine untere Wand (38), Innenwände (28, 39) enthält, wobei die Wände (25, 28, 38, 39) im wesentlichen ringförmige Kanäle definieren, durch die der Kraftstoff (15) absteigt, und Durchbohrungen (26, 126) für den Durchgang und/oder mindestens horizontalen Austausch der absteigenden kalten gasförmigen Fluide des festen Kraftstoffs (15) und der aufsteigenden heißen gasförmigen Fluide, die in koordinierten Zwischenräumen (27, 34) zirkulieren, umfassen.

2. Verfahren nach Anspruch 1, **dadurch gekennzeichnet, daß** ein gewünschter Teil der Abgase (56), die durch die Verbrauchervorrichtung (54) hergestellt werden, in den Vergaser (10) zusammen mit Verbrennungsmittelfluiden (58-59) zurückgeführt wird, um die Heizkraft des hergestellten brennbaren Gases (45) zu verringern.

3. Verfahren nach Anspruch 1, **dadurch gekennzeichnet, daß** mindestens ein Teil der in dem brennbaren Gas (45) enthaltenen Wärme in mindestens einem Austauscher (46, 48) außerhalb des Vergasers (10) eingesetzt wird, um Verbrennungsmittelluft (58) zu erhitzen, bevor die Luft (58) in den Vergaser (10) eingeführt wird.

4. Verfahren nach Anspruch 1, **dadurch gekennzeichnet, daß** mindestens ein Teil der Wärme des brennbaren Gases (45) in einem Austauscher (46, 47) außerhalb des Vergasers (10) eingesetzt wird, um Wasserdampf (59) zu erhitzen, bevor der Wasserdampf (59) in den Vergaser (10) eingeführt wird.

5. Verfahren nach Anspruch 1, **dadurch gekennzeichnet, daß** mindestens ein Teil der Wärme der Abgase (56) der Verbrauchervorrichtung (54) in einem Wärmeaustauscher (57) eingesetzt wird, um die festen Kraftstoffe (5) zu entwässern, bevor sie in den Vergaser (10) eingeführt werden.

6. Verfahren nach Anspruch 1, **dadurch gekennzeichnet, daß** mindestens ein Teil der Wärme der Abgase (56) der Verbrauchervorrichtung (54) in einem Wärmeaustauscher (62) eingesetzt wird, um Wasserdampf (59) zu erhalten, der in den Vergaser (10) eingeführt wird.

7. Verfahren nach Anspruch 1, **dadurch gekennzeichnet, daß** mindestens ein Teil der Wärme der Abgase (56) der Verbrauchervorrichtung (54) in einem Wärmeaustauscher (70) eingesetzt wird, um flüssigen Kraftstoff (73) in dampfförmigen Kraftstoff (74) zu überführen, der dann in den Vergaser (10) eingeführt wird.

8. Verfahren nach Anspruch 1, **dadurch gekennzeichnet, daß** mindestens ein Teil der Wärme der Abgase (56) der Verbrauchervorrichtung (54) in einer Trocknungsvorrichtung (65) eingesetzt wird, um den festen Kraftstoff (15), der in den Vergaser (10) eingeführt werden soll, vorher zu trocknen.

9. Verfahren nach einem der vorstehenden Ansprüche, **dadurch gekennzeichnet, daß** es die Verwendung mindestens einer Waschvorrichtung (49) bereitstellt, um das hergestellte brennbare Gas (45) zu reinigen, bevor es in die Verbrauchervorrichtung (54) eingespeist wird.

10. Verfahren nach Anspruch 9, **dadurch gekennzeichnet, daß** die Waschvorrichtung (49) eine Vorrichtung (50) enthält, die granuliertes Calciumcarbonat (CaCO₃) enthält, das mit Wasser (63) zum Waschen des Gases (45) besprüht wird, wobei das Wasser (63) von einem Turm (51) mit duschenartigen Kühlung bereitgestellt wird.

11. Verfahren nach Anspruch 10, **dadurch gekennzeichnet, daß** der Turm (51) mit duschenartiger Kühlung die Reinigung des Waschwassers mit Hilfe von mechanischen und/oder chemischen Systemen bereitstellt.

12. Verfahren nach einem vorstehenden Ansprüche, **gekennzeichnet durch** die Verwendung von Mitteln (64) zur Entfernung von Kesselstein aus mindestens dem Wasser (63), das in Dampf (59) überführt werden soll.

13. Verfahren nach einem vorstehenden Ansprüche, **dadurch gekennzeichnet, daß** die Abgase (56), bevor sie in die Atmosphäre entlassen werden, gekühlt, gereinigt und mittels einer Reinigungs-Kühlungsvorrichtung (66) und/oder einer Vorrichtung zur Entfernung von Gerüchen (67) von unangenehmen Gerüchen und umweltverschmutzenden Substanzen befreit werden.

14. Apparat zur Herstellung von brennbarem Gas (45), welche von festen Kraftstoffen (15), wie festen Haushalts- und/oder Industrieabfall, Holzresten und/oder -schnitzeln, Biomasse im allgemeinen, Kohlenstoffen, Torf oder Lignit, ausgeht, wobei der Apparat mindestens einen Vergaser und mindestens eine Verbrauchervorrichtung (54), bestehend aus einem Dampfmotor oder Boiler, des brennbaren Gases (45) enthält, wobei der Vergaser die Beladungsmittel (12) für den festen Kraftstoff (15) in einer oberen Beladungszone (23) enthält, die über der mindestens einen oberen Trockendestillationszone (24) und mindestens einer unteren Reduktionszone (36), die mit einer benachbarten Oxidationszone (35) in Verbindung steht, angeordnet ist, wobei die Vorrichtung **dadurch gekennzeichnet ist, daß** der Vergaser im Inneren mindestens ein Paar von Innenwänden (28, 39) enthält, welche den festen Kraftstoff (15) aufnehmen sollen, die Wände innerhalb eines Paares von äußeren Wänden, jeweils die obere äußere Wand (25) und die untere äußere Wand (38), angeordnet sind, die Wände (25, 28, 38, 39) im wesentlichen ringförmige Kanäle definieren, durch die der Kraftstoff (15) absteigt, und Durchbohrungen (26, 126) für den Durchgang und/oder mindestens horizontalen Austausch der absteigenden kalten gasförmigen Fluide des festen Kraftstoffs (15) und der aufsteigenden heißen gasförmigen Fluide enthält, die in koordinierten Zwischenräumen (27, 34) zirkulieren, die mindestens außerhalb der äußeren Wände (25, 38) definiert sind, und **dadurch gekennzeichnet, daß** außerhalb des Vergasers (10) der Apparat Wärmeaustauscher (46, 47, 48) enthält, die mindestens einen Teil der Wärme des hergestellten brennbaren Gases (45) und der Wärme der Abgase der Verbrauchervorrichtung (54) wiedergewinnen können, um mindestens Verbrennungsmittelfluide (58, 59), die in den Vergaser (10) eingeführt werden sollen, zu erhitzen.

15. Apparat nach Anspruch 14, **dadurch gekennzeichnet, daß** die äußeren Wände (25, 38) des Vergasers (10) einen Raum definieren, das wie ein abgeschnittener Konus geformt ist, dessen größerer Boden in Richtung des Bodens des Vergasers (10) gerichtet ist, und die Innenwände (28, 39) geeignet sind, um eine zentrale Achse (30) des Vergasers (10) zu rotieren.

16. Apparat' nach Anspruch 14, **dadurch gekennzeichnet, daß** mindestens die unteren äußeren Wände (38), die in der Nähe der Reduktionszone (36) angeordnet sind, in einer Nickellegierung eingebettet sind oder mindestens im Innern mit Nickel ausgelegt sind.

17. Apparat nach Anspruch 14, **dadurch gekennzeichnet, daß** die untere Innenwand (39) und die obere Innenwand (28) des Vergasers (10) fest sind und mit einem drehbaren Schaft (40) ausgestattet sind.

18. Apparat nach einem der Ansprüche 14 bis 17, **dadurch gekennzeichnet, daß** der Vergaser (10) im unteren Teil ein Scheibenelement (41) enthält, um Asche aufzunehmen und zu entfernen, wobei das Scheibenelement (41) in Zusammenwirkung mit mindestens einem Schaufelelement (72) für die kontrollierte Entfernung der Asche angeordnet ist.

19. Apparat nach Anspruch 18, **dadurch gekennzeichnet, daß** der Vergaser (10) eine Sensorvorrichtung (43) enthält, um die Temperatur mindestens der Asche, deren Entfernung sie steuert, überwacht.

20. Apparat nach einem der Ansprüche 14 bis 19, **dadurch gekennzeichnet, daß** der Vergaser (10) gasdichte Beladungsmittel vom Typ einer dosierenden Schnecke (13) enthält.

21. Apparat nach Anspruch 20, **dadurch gekennzeichnet, daß** die dosierende Schnecke (13) in einem Zylinder (14) enthalten ist, der bezüglich des rechten Winkels der Achse (30) des Vergasers (10) nach unten geneigt ist.

22. Apparat nach einem der Ansprüche 20 oder 21, **dadurch gekennzeichnet, daß** die dosierende Schnecke (13) von einem Motor (21) angetrieben werden, der durch einen Sensor, der das Niveau (22) überwacht, gesteuert wird.

23. Apparat nach Anspruch 14, **dadurch gekennzeichnet, daß** er außerhalb des Vergasers (10) mindestens einen Wärmeaustauscher (57) enthält, um den Kraftstoff (15) dadurch vorher zu entwässern, daß die heißen Abgase (56), die durch die Verbrauchervorrichtung (54) hergestellt werden, in den Austauscher (57) geleitet werden.

24. Apparat nach Anspruch 14, **dadurch gekennzeichnet, daß** er außerhalb des Vergasers (10) mindestens einen Wärmeaustauscher (64) enthält, um Wasserdampf (59) dadurch zu erhalten, daß heiße Abgase (56), die durch die Verbrauchervorrichtung (54) hergestellt werden, in den Austauscher (62) geleitet werden.

25. Apparat nach Anspruch 14, **dadurch gekennzeichnet, daß** er außerhalb des Vergasers (10) mindestens einen Wärmeaustauscher (70) enthält, um flüssige Kraftstoffe (73) in verdampfte Kraftstoffe (74) dadurch zu überführen, daß die heißen Abgase (56), die durch die Verbrauchervorrichtung (54) hergestellt werden, in den Austauscher (70) geleitet werden.

26. Apparat nach Anspruch 14, **dadurch gekennzeichnet, daß** er außerhalb des Vergasers (10) mindestens eine Waschvorrichtung (49) enthält, um das brennbare Gas (45) zu waschen, um es von Pulvern, die in Suspension enthalten sind, sauren Substanzen und anderen verschmutzenden Elementen zu reinigen.

27. Apparat nach Anspruch 26, **dadurch gekennzeichnet, daß** die Waschvorrichtung (49) mindestens eine Vorrichtung (50) enthält, welche mit Calciumcarbonat wäscht.

## Revendications

1. Procédé pour la gazéification de combustibles solides (15) pour produire un gaz combustible (45) à partir de déchets urbains et/ou industriels solides, de morceaux et copeaux de bois, de biomasses en général, de charbon, de tourbe ou de lignite, ledit procédé utilisant au moins un réacteur de gazéification associé au moins à un dispositif (54), constitué d'un moteur ou d'une chaudière à vapeur, capable d'utiliser le gaz combustible (45) produit, le procédé étant **caractérisé en ce qu'**il prévoit d'effectuer, en dehors dudit réacteur de gazéification (10), en alternance ou conjointement, au moins une procédure de récupération thermique d'au moins une partie de la chaleur dudit gaz combustible (45) sortant du réacteur de gazéification (10), d'au moins une partie de la chaleur des gaz d'échappement produits par ledit dispositif utilisateur (54), ou d'au moins une partie de la chaleur générée par les parties mécaniques dudit dispositif utilisateur (54), le procédé étant également **caractérisé en ce qu'**il prévoit une distillation préalable, au moyen d'un préchauffage, des parties volatiles du combustible solide (15), immédiatement après l'introduction du combustible solide (15) dans ledit réacteur de gazéification (10), pour produire un gaz combustible sans aucune substance goudronneuse, et il prévoit d'inclure, dans la partie inférieure dudit réacteur de gazéification (10), des moyens catalyseurs constitués de nickel, capables d'assister la formation de gaz méthane (45), et **en ce que**, dans une partie supérieure (24) du réacteur de gazéification (10), il prévoit un échange tant dans la direction verticale que dans la direction horizontale, entre les fluides froids descendants et/ou les distillats des parties volatiles présentes dans le matériau combustible (15) chargé par le dessus et les fluides chauds ascendants provenant des parties intermédiaire et inférieure du réacteur de gazéification (10), de façon à obtenir dans ladite partie supérieure (24), une distillation sèche, en raison d'un chauffage sans combustion des parties volatiles dudit combustible solide (15), ledit réacteur de gazéification (10) comprenant des parois intérieures (28, 39) contenues à l'intérieur de parois extérieures, respectivement supérieure (25) et inférieure (38), lesdites parois (25, 28, 38, 39) définissant des canaux essentiellement annulaires à travers lesquels le combustible (15) descend, et comprenant des trous débouchants (26, 126) pour le passage et/ou au moins l'échange horizontal des fluides gazeux froids descendants du combustible solide (15) et des fluides gazeux chauds ascendants circulant dans des espaces reliés entre eux (27, 34).

2. Procédé selon la revendication 1, **caractérisé en ce qu'**il prévoit qu'une partie souhaitée des gaz d'échappement (56) produits par ledit dispositif utilisateur (54) soit réintroduite dans le réacteur de gazéification (10), en même temps que des fluides comburants (58-59), afin de réduire la puissance calorifique du gaz combustible (45) produit.

3. Procédé selon la revendication 1, **caractérisé en ce qu'**au moins une partie de la chaleur du gaz combustible (45) est utilisée, dans au moins un échangeur (46, 48), à l'extérieur du réacteur de gazéification (10), pour chauffer l'air comburant (58) avant que ledit air (58) soit envoyé à l'intérieur du réacteur de gazéification (10).

4. Procédé selon la revendication 1, **caractérisé en ce qu'**au moins une partie de la chaleur du gaz combustible (45) est utilisée, dans un échangeur (46, 47), à l'extérieur du réacteur de gazéification (10), pour chauffer la vapeur d'eau (59) avant que ladite vapeur d'eau (59) soit envoyée à l'intérieur du réacteur de gazéification (10).

5. Procédé selon la revendication 1, **caractérisé en ce qu'**au moins une partie de la chaleur des gaz d'échappement (56) dudit dispositif utilisateur (54) est utilisée, dans un échangeur de chaleur (57), pour préalablement déshydrater lesdits combustibles solides (15) avant qu'ils soient envoyés à l'intérieur du réacteur de gazéification (10).

6. Procédé selon la revendication 1, **caractérisé en ce qu'**au moins une partie de la chaleur des gaz d'échappement (56) dudit dispositif utilisateur (54) est utilisée, dans un échangeur de chaleur (62), pour obtenir de la vapeur d'eau (59) à envoyer à l'intérieur du réacteur de gazéification (10).

7. Procédé selon la revendication 1, **caractérisé en ce qu'**au moins une partie de la chaleur des gaz d'échappement (56) dudit dispositif utilisateur (54) est utilisée, dans un échangeur de chaleur (70), pour transformer des combustibles liquides (73) en combustible vaporisé (74) à envoyer à l'intérieur du réacteur de gazéification (10).

8. Procédé selon la revendication 1, **caractérisé en ce qu'**au moins une partie de la chaleur des gaz d'échappement (56) dudit dispositif utilisateur (54) est utilisée, dans un dispositif de séchage (65), pour effectuer un séchage préalable du combustible solide (15) à envoyer à l'intérieur du réacteur de gazéification (10).

9. Procédé selon l'une quelconque des revendications précédentes, **caractérisé en ce qu'**il prévoit d'utiliser au moins un dispositif de lavage (49) pour purifier le gaz combustible (45) produit, avant qu'il soit envoyé à l'intérieur du dispositif utilisateur (54).

10. Procédé selon la revendication 9, **caractérisé en ce que** le dispositif de lavage (49) comprend un dispositif (50) contenant du carbonate de calcium granulé (CaCO₃), pulvérisé avec de l'eau (63) pour laver le gaz (45), l'eau (63) étant fournie par une tour de refroidissement par arrosage (51).

11. Procédé selon la revendication 10, **caractérisé en ce que** la tour de refroidissement par arrosage (51) prévoit de purifier l'eau de lavage au moyen de systèmes mécaniques et/ou chimiques.

12. Procédé selon l'une quelconque des revendications précédentes, **caractérisé en ce qu'**il prévoit d'utiliser des moyens anti-tartre (64), au moins pour l'eau (63) qui doit être transformée en vapeur (59).

13. Procédé selon l'une quelconque des revendications précédentes, **caractérisé en ce que** les gaz d'échappement (56), avant d'être expulsés dans l'atmosphère, sont refroidis, purifiés et dépossédés des odeurs désagréables et des substances polluantes au moyen d'un dispositif de purification et de refroidissement (66) et/ou d'un dispositif d'élimination des odeurs (67).

14. Appareil pour la production de gaz combustible (45) à partir de combustibles solides (15), tels que des déchets urbains et/ou industriels solides, des morceaux et copeaux de bois, des biomasses en général, du charbon, de la tourbe ou de la lignite, ledit appareil comprenant au moins un réacteur de gazéification et au moins un dispositif (54), constitué d'un moteur ou d'une chaudière à vapeur, utilisateur dudit gaz combustible (45), le réacteur de gazéification comprenant des moyens de chargement (12) pour le combustible solide (15), dans une zone de chargement supérieure (23) située au-dessus d'au moins une zone de distillation sèche supérieure (24) et d'au moins une zone de réduction inférieure (36) coopérant avec une zone d'oxydation contiguë (35), le dispositif étant **caractérisé en ce que** ledit réacteur de gazéification comprend au moins une paire de parois intérieures (28, 39) pour contenir le combustible solide (15), les parois étant contenues à l'intérieur d'une paire de parois extérieures, respectivement la paroi extérieure supérieure (25) et la paroi extérieure inférieure (38), lesdites parois (25, 28, 38, 39) définissant des canaux essentiellement annulaires à travers lesquels le combustible (15) descend, et comprenant des trous débouchants (26, 126) pour le passage et/ou au moins l'échange horizontal des fluides gazeux froids descendants du combustible solide (15) et des fluides gazeux chauds ascendants circulant dans des espaces reliés entre eux (27, 34), définis au moins à l'intérieur desdites parois extérieures (25, 38), et **en ce que**, à l'extérieur dudit réacteur de gazéification (10), l'appareil comprend des échangeurs de chaleur (46, 47, 48) capables de récupérer au moins une partie de la chaleur du gaz combustible (45) produit et des gaz d'échappement dudit dispositif utilisateur (54), pour chauffer au moins des fluides comburants (58, 59) à envoyer audit réacteur de gazéification (10).

15. Appareil selon la revendication 14, **caractérisé en ce que** lesdites parois de confinement extérieures (25, 38) dudit réacteur de gazéification (10) définissent un volume en forme de tronc de cône dont la base la plus grande est dirigée vers le fond du réacteur de gazéification (10), et lesdites parois de confinement intérieures (28, 39) peuvent être entraînées en rotation autour d'un axe central (30) du réacteur de gazéification (10).

16. Appareil selon la revendication 14, **caractérisé en ce qu'**au moins lesdites parois de confinement extérieures inférieures (38) placées à proximité de la zone de réduction (36) sont constituées d'un alliage de nickel, ou revêtues de nickel, au moins à l'intérieur.

17. Appareil selon la revendication 14, **caractérisé en ce que** la paroi intérieure inférieure (39) et la paroi intérieure supérieure (28) dudit réacteur de gazéification (10) sont solidaires d'un arbre rotatif (40).

18. Appareil selon l'une quelconque des revendications 14 à 17 comprises, **caractérisé en ce que** ledit réacteur de gazéification (10) comprend, au niveau de la partie inférieure, un élément de disque (41) servant à récupérer et expulser les cendres, l'élément de disque (41) étant placé en coopération avec au moins un élément de lame (72) pour décharger les cendres sous commande.

19. Appareil selon la revendication 18, **caractérisé en ce que** ledit réacteur de gazéification (10) comprend un dispositif capteur (43) pour surveiller la température d'au moins les cendres dont le déchargement est gouverné.

20. Appareil selon Tune quelconque des revendications 14 à 19 comprises, **caractérisé en ce que** ledit réacteur de gazéification (10) comprend des moyens de chargement étanches aux gaz, du type vis de dosage (13).

21. Appareil selon la revendication 20, **caractérisé en ce que** les moyens de vis de dosage (13) sont contenus dans un cylindre (14) incliné vers le bas par rapport à l'angle droit de l'axe (30) du réacteur de gazéification (10).

22. Appareil selon l'une quelconque des revendications 20 à 21 comprises, **caractérisé en ce que** les moyens de vis de dosage (13) sont entraînés par des moyens formant moteur (21) gouvernés par des moyens de détection pour surveiller le niveau (22).

23. Appareil selon la revendication 14, **caractérisé en ce que**, à l'extérieur du réacteur de gazéification (10), il comprend au moins un échangeur de chaleur (57) servant à déshydrater préalablement le combustible (15) par l'envoi des gaz d'échappement chauds (56) produits par le dispositif utilisateur (54) dans ledit échangeur (57).

24. Appareil selon la revendication 14, **caractérisé en ce que**, à l'extérieur du réacteur de gazéification (10), il comprend au moins un échangeur de chaleur (64) servant à obtenir de la vapeur d'eau (59) par l'envoi des gaz d'échappement chauds (56) produits par le dispositif utilisateur (54) dans ledit échangeur (62).

25. Appareil selon la revendication 14, **caractérisé en ce que**, à l'extérieur du réacteur de gazéification (10), il comprend au moins un échangeur de chaleur (70) servant à transformer les combustibles liquides (73) en combustibles vaporisés (74) par l'envoi des gaz d'échappement chauds (56) produits par le dispositif utilisateur (54) dans ledit échangeur (70).

26. Appareil selon la revendication 14, **caractérisé en ce que**, à fextérieur du réacteur de gazéification (10), il comprend au moins un dispositif de lavage (49) pour laver ledit gaz combustible (45), afin de le purifier des poudres présentes en suspension, des substances acides et d'autres éléments polluants.

27. Appareil selon la revendication 26, **caractérisé en ce que** ledit dispositif de lavage (49) comprend au moins un dispositif (50) qui effectue un lavage au carbonate de calcium.
